# EUROPEAN PATENT APPLICATION

(11) **EP 3 988 156 A1**
(43) Date of publication of application: **27.04.2022**
(21) Application number: 19933517.5
(22) Date of filing: 21.06.2019
(51) Int. Cl.: A61M 25/09

(54) **GUIDE WIRE**

(71) Applicant: Asahi Intecc Co., Ltd., Seto-shi, Aichi 489-0071 (JP)
(72) Inventor: TSUGE Kenta, Seto-shi, Aichi 489-0071 (JP)
(74) Representative: Winter, Brandl - Partnerschaft mbB
(86) International application number: PCT/JP2019/024804
(87) International publication number: WO 2020/255400

(57) **Abstract**

The present invention provides a guide wire which prevents a proximal end portion of a U-shaped curved portion formed at a distal end portion of the guide wire from proceeding beyond an expanded diameter portion to the proximal side, and prevents plastic deformation from occurring in the core shaft at the expanded diameter portion.

The guide wire 1 includes: a core shaft 11 having a first expanded diameter portion 111 which expands in diameter toward the proximal end side, and a first extended portion 112 having a cylindrical shape or truncated cone shape that becomes thinner toward the distal end side; a first coil body 21 wound so as to cover at least a portion of the core shaft 11; and a distal end fixing portion 41 at which the distal end of the core shaft 11 and the distal end of the first coil body 21 are fixed to each other.

## Description

### TECHNICAL FIELD

The present invention relates to a guide wire.

### BACKGROUND ART

When treating a site such as a constricted part that has formed inside a blood vessel, a guide wire is inserted prior to a treatment device such as a catheter.

In order to enable the guide wire to proceed through complicated, curved blood vessels and reach the treatment site with certainty, the guide wire is required to have a flexible distal end portion, and torquability so that rotation operations performed by hand can be propagated with certainty to the distal end portion.

As an example of such a guide wire, a guide wire provided with a core wire having a tapered portion, and a coil wound so as to cover the core wire is known (for example, see Patent Literature 1).

According to the guide wire described above, it is possible to obtain flexibility of the distal end portion due to the core wire having a reduced diameter and a high torquability due to the coil, and therefore, smooth insertion into a treatment site inside a blood vessel can be expected.

### CITATION LIST

### Patent Literature

[Patent Literature 1] JPH07-227429

### SUMMARY OF INVENTION

TECHNICAL PROBLEM[0007] Although the conventional guide wire described above can be expected to provide an improvement in terms of flexibility and torquability, the proximal end portion of a U-shaped curved portion formed on the distal end portion of the guide wire may proceed beyond the tapered portion of the core wire to the proximal side, or the core wire at this part may become plastically bent, which may hinder the smooth insertion into a blood vessel.

The present invention has been made based on the above circumstances, and an object of the present invention is to provide a guide wire with which it is possible to prevent a proximal end portion of a U-shaped curved portion formed on a distal end portion of a guide wire from proceeding beyond an expanded diameter portion to the proximal end side, and prevent plastic deformation from occurring in the core shaft at the expanded diameter portion.

### SOLUTION TO PROBLEM

Some aspects of the present disclosure include:
(1) A guide wire that includes a core shaft having a first expanded diameter portion which expands in diameter toward a proximal end side and a first extended portion having a cylindrical shape or a truncated cone shape that becomes thinner toward a distal end side, which is provided adjacent to the first expanded diameter portion so as to extend along a longitudinal axis direction, a first coil body wound so as to cover at least a portion of the core shaft, and a distal end fixing portion at which a distal end of the core shaft and a distal end of the first coil body are fixed to each other; wherein a gradient of a proximal end of the first expanded diameter portion is greater than a gradient of a distal end of the first expanded diameter portion, an average gradient between a distal end and a proximal end of the first expanded diameter portion is greater than a gradient of the first extended portion, a gradient of the first expanded diameter portion increases continuously toward a proximal end side, and a proximal end of the first coil body is not fixed to the first expanded diameter portion, but is fixed to the first extended portion;
(2) The guide wire according to (1) above, wherein the gradient of the first expanded diameter portion gradually changes along a longitudinal axis direction;
(3) The guide wire according to (2) above, wherein a gradient at the distal end of the first expanded diameter portion is zero, and the proximal end of the first expanded diameter portion stands perpendicular to a longitudinal axis;
(4) The guide wire according to any one of (1) to (3) above, wherein the core shaft includes a second expanded diameter portion which expands in diameter toward a proximal end side, and the first extended portion is arranged between the first expanded diameter portion and the second expanded diameter portion;
(5) The guide wire according to (4) above, wherein a gradient of the second expanded diameter portion is greater than the gradient of the first extended portion, and a gradient of the proximal end of the second expanded diameter portion is greater than a gradient of a distal end of the second expanded diameter portion;
(6) The guide wire according to (4) above, wherein a gradient of the second expanded diameter portion is greater than a gradient of the first extended portion, and is constant along a longitudinal axis direction; and
(7) The guide wire according to any one of (1) to (3) above, including a second coil body wound so as to cover the first coil body, wherein the core shaft includes the first extended portion disposed on a distal end side of the first expanded diameter portion, and a second extended portion having a cylindrical shape or a truncated cone shape that becomes thinner toward a distal end side, and which is provided on a proximal end side of the first expanded diameter portion and extends along a longitudinal axis direction, and a proximal end of the second coil body is not fixed to the first expanded diameter portion, but is fixed to the second extended portion.

In the present specification, the "distal side" is a direction along the longitudinal axis direction of the guide wire, and refers to the direction in which the distal end fixing portion is located with respect to the first coil body. The "proximal end side" is a direction along the longitudinal axis direction of the guide wire, and refers to the opposite direction to the distal end side. In addition, the "distal end" refers to the end portion of any member or part on the distal end side, and the "proximal end" refers to the end portion of any member or part on the proximal end side. Furthermore, the term "gradient" refers to the amount of increase in the radius of the core shaft per unit distance in the longitudinal axis direction. The "longitudinal axis" refers to the central axis of the core shaft, and the "longitudinal axis direction" refers to a direction along the longitudinal axis.

### ADVANTAGEOUS EFFECTS OF INVENTION

The present invention is capable of providing a guide wire with which it is possible to prevent a proximal end portion of a U-shaped curved portion formed on a distal end portion of a guide wire from proceeding beyond an expanded diameter portion to the proximal end side, and prevent plastic deformation from occurring in the core shaft at the expanded diameter portion.

### BRIEF DESCRIPTION OF DRAWINGS

FIG. 1A is a schematic side view showing a first embodiment of the present invention.
FIG. 1B is a schematic side view showing an example of FIG. 1A when in use.
FIG. 2Ais a partially enlarged schematic side view showing a preferable example of the first embodiment.
FIG. 2B is a schematic cross-sectional view showing enlarged a first expanded diameter portion of the core shaft in FIG. 2A.
FIG. 3Ais a partially enlarged schematic side view showing a modification of FIG. 1A.
FIG. 3B is a partially enlarged schematic side view showing a modification of FIG. 1A.
FIG. 4 is a partially enlarged schematic side view showing a second embodiment of the present invention.
FIG. 5 is a partially enlarged schematic side view showing a modification of FIG. 4.
FIG. 6 is a partially enlarged schematic side view showing a third embodiment of the present invention.

### DESCRIPTION OF EMBODIMENTS

The guide wire includes: a core shaft having a first expanded diameter portion which expands in diameter toward a proximal end side, and a first extended portion having a cylindrical shape or a truncated cone shape that becomes thinner toward a distal end side, and which is provided adjacent to the first expanded diameter portion so as to extend along a longitudinal axis direction; a first coil body wound so as to cover at least a portion of the core shaft; and a distal end fixing portion at which a distal end of the core shaft and a distal end of the first coil body are fixed to each other; and is characterized in that a gradient of a proximal end of the first expanded diameter portion is greater than a gradient of a distal end of the first expanded diameter portion, an average gradient between a distal end and a proximal end of the first expanded diameter portion is greater than a gradient of the first extended portion, a gradient of the first expanded diameter portion increases continuously toward a proximal end side, and a proximal end of the first coil body is not fixed to the first expanded diameter portion, but is fixed to the first extended portion.

In the present specification, the "distal end portion" refers to a part which includes the distal end of any member or part, which extends from the distal end to a part in the middle toward the proximal end side of the member or the like. The "proximal end portion" refers to a portion which includes the proximal end of any member or part, which extends from the proximal end to a part in the middle toward the distal end side of the member or the like.

First to third embodiments of the present invention will be described below with reference to the drawings, but the present invention is not limited only to the embodiments described in the drawings. Note that the dimensions of the guide wire illustrated in the drawings are dimensions shown for the purpose of facilitating an understanding of the implementation details, and do not correspond to the actual dimensions.

### [First Embodiment]

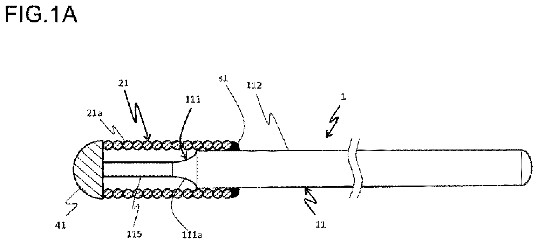

FIG. 1A is a schematic side view showing a first embodiment of the present invention. As illustrated in FIG. 1A, a guide wire 1 is schematically composed of a core shaft 11, a first coil body 21, and a distal end fixing portion 41.

The core shaft 11 is a shaft in which the distal end portion has a diameter which reduces stepwise toward the distal end side. For example, the core shaft 11 is configured to have a first expanded diameter portion 111, a first extended portion 112, and a small diameter portion 115.

The first expanded diameter portion 111 is a part of the core shaft 11 in which the diameter expands toward the proximal end side. The gradient of the proximal end of the first expanded diameter portion 111 is greater than the gradient of the distal end of the first expanded diameter portion 111, the average gradient between the distal end and the proximal end of the first expanded diameter portion 111 is greater than the gradient of the first extended portion 112, and the gradient of the first expanded diameter portion 111 increases continuously toward the proximal end side. Specifically, the first expanded diameter portion 111 may adopt, for example, a structure in which the gradient gradually changes along the longitudinal axis direction (a structure in which an outer peripheral surface 111a of the first expanded diameter portion 111 has a curved shape in a cross-section including the longitudinal axis); a structure that uses a plurality of truncated cones having different gradients that are joined such that the end surfaces coincide with each other, and which are arranged such that the truncated cone having the smaller gradient is on the distal end side of the first expanded diameter portion, and the truncated cone having the larger gradient is on the proximal end side of the first expanded diameter portion; or structure that combines these structures.

The first extended portion 112 is a part of the core shaft 11 having a cylindrical shape or a truncated cone shape that becomes thinner toward the distal end side, and which is provided adjacent to the first expanded diameter portion 111 so as to extend along the longitudinal axis direction.

Here, it is preferable that the gradient of the first expanded diameter portion 111 described above gradually changes along the longitudinal axis direction. As a result, the rigidity of the core shaft 11 in the longitudinal axis direction can be smoothly changed along the longitudinal axis direction, and plastic deformation of the guide wire 1 at the first expanded diameter portion 111 can be prevented.

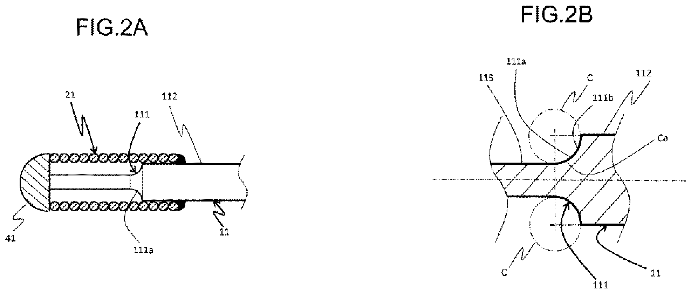

Furthermore, it is preferable that the gradient of the distal end of the first expanded diameter portion 111 is zero, and the proximal end of the first expanded diameter portion 111 stands perpendicular to the longitudinal axis (the surface of the proximal end of the first expanded diameter portion is perpendicular to the longitudinal axis). An example of such a first expanded diameter portion 111 is a structure in which a contour line 111b of the outer peripheral surface 111a of the first expanded diameter portion in a cross-section including the axis L is an arc Ca equivalent to one quarter of the circumference C (see FIG. 2A and FIG. 2B). In FIG. 2B, the double-dotted line represents the circumference C shown hypothetically. As a result, the rigidity of the core shaft 11 in the longitudinal axis direction can be smoothly and monotonically changed, and plastic deformation of the guide wire 1 at the first expanded diameter portion 111 can be effectively prevented.

In the present embodiment, the core shaft 11 is configured such that the gradient of the first expanded diameter portion 111 gradually changes along the longitudinal axis direction (the outer peripheral surface 111a is curved), and the cylindrical first extended portion 112 (constant outer diameter) extends toward the proximal end side so as to be continuous with the proximal end of the first expanded diameter portion 111. Furthermore, the distal end of the first expanded diameter portion 111 is provided with a cylindrical small diameter portion 115 (constant outer diameter) extending toward the distal end side.

In terms of the dimensions of the core shaft 11 in the longitudinal axis direction, the total length is typically 1,800 to 3,000 mm, and the first expanded diameter portion 111 is 1 mm to 3 mm. The outer diameter of the core shaft 11 is typically 0.25 mm to 0.46 mm at the first extended portion 112.

The first coil body 21 is a helical (coiled) member wound so as to cover at least a portion of the core shaft 11. The first coil body 21 may adopt, for example, a structure in which a wire 21a is wound to form a coil shape (see FIG. 1A), or a structure in which a cylindrical member is subjected to slit processing or the like to form a coil shape (not illustrated). When the wire 21a is used for the first coil body 21, it is possible for the wire 21a to use one or more solid wires, or one or more twisted wires. Note that, a solid wire refers to a single wire, and a twisted wire refers to a bundled group of wires formed by twisting a plurality of single wires with each other in advance.

The diameter of the wire 21a (solid wire or twisted wire) constituting the first coil body 21 is typically 0.01 to 0.10 mm.

In terms of the wire material constituting the first coil body 21, from the perspective of ensuring the flexibility of the guide wire 1 and imparting antithrombogenicity and biocompatibility, it is possible to use, for example, stainless steel as SUS316; a superelastic alloy such as an Ni-Ti alloy; or a radiopaque metal such as platinum or tungsten.

The proximal end of the first coil body 21 is not fixed to the first expanded diameter portion 111, but is fixed to the first extended portion 112 (joint part s1 in FIG. 1A). In terms of the method of fixing the proximal end of the first coil body 21 with the first extended portion 112, for example, it is possible to use a method that joins by means of a brazing method using a brazing material. Examples of the brazing material include brazing metals such as an Sn-Pb alloy, a Pb-Ag alloy, an Sn-Ag alloy, and an Au-Sn alloy.

The distal end fixing portion 41 is a part in which the distal end of the core shaft 11 and the distal end of the first coil body 21 are integrally fixed. Specifically, for example, the distal end fixing portion 41 is formed so that the distal end portion forms a substantially hemispherical shape which is curved in a convex shape toward the distal end side. As a result, the resistance can be reduced when the guide wire 1 moves forward in a blood vessel, and the guide wire 1 can be smoothly inserted.

Examples of the method of forming the distal end fixing portion 41 that can be adopted include a method that forms the distal end fixing portion 41 by melting and molding a portion of the members constituting the core shaft 11 and/or the first coil body 21, and a method that forms the distal end fixing portion 41 by joining the core shaft 11 and the first coil body 21 using a brazing material, and then molding the brazing material. Examples of the brazing material include the same materials as those described above in the method of fixing the proximal end of the first coil body 21.

Next, the manner in which the guide wire 1 is used will be explained. First, the guide wire 1 is inserted from the distal end into a blood vessel, and the distal end portion is pushed forward by operating the guide wire 1 exposed outside the body. At this time, in order to improve the operability and the like, the distal end portion of the guide wire 1 may be intentionally bent prior to insertion to the blood vessel, or a U-shaped curved portion may be formed by an unintentional bending force while proceeding through the blood vessel.

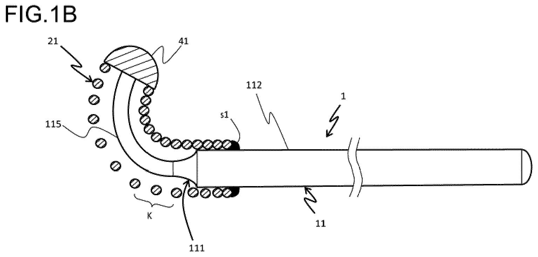

Here, although a proximal end portion K of the curved portion described above (see FIG. 1B) typically proceeds (moves) to the proximal end side as the guide wire 1 is pushed into the blood vessel, it is difficult for the proximal end portion K of the curved portion to proceed beyond the first expanded diameter portion 111 to the proximal end side, and because the core shaft 11 does not easily become plastically bent at the first expanded diameter portion 111, the guide wire 1 can be efficiently inserted up to the treatment site due to the excellent operability.

Then, after the distal end portion of the guide wire 1 reaches the treatment site, the proximal end of the guide wire 1 is inserted from the distal end of a medical device such as a catheter (not illustrated) and into an inner cavity of the device, and once the guide wire 1 protrudes from the proximal end of the medical device, the medical device is pushed forward along the guide wire 1. Next, once the medical device reaches the treatment site, various treatments can be performed using the medical device. Then, after completion of the treatments, the series of procedures is completed by withdrawing the medical device and the guide wire 1 from the blood vessel.

Because the guide wire 1 has the configuration described above, it is possible to prevent the proximal end portion K of the U-shaped curved portion formed on the distal end portion of the guide wire 1 from proceeding beyond the first expanded diameter portion 111 to the proximal end side, and plastic deformation of the core shaft 11 at the first expanded diameter portion 111 can be prevented, and therefore, the guide wire 1 can be smoothly operated in a blood vessel and efficiently inserted up to the treatment site. Here, because the proximal end of the first coil body 21 is not fixed to the first expanded diameter portion 111, the rigidity difference in the longitudinal axis direction can be made large before and after the first expanded diameter portion 111, and this large rigidity difference may prevent the proximal end portion K of the curved portion from proceeding. In addition, because the gradient of the first expanded diameter portion 111 is greater than the gradient of the first extended portion 112, and the gradient of the proximal end of the first expanded diameter portion 111 is greater than the gradient of the distal end of the first expanded diameter portion 111, the rate of change in the rigidity of the first expanded diameter portion 111 in the longitudinal axis direction can be made more gradual, which may prevent plastic deformation of the first expanded diameter portion 111.

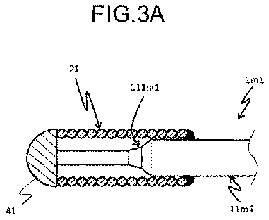

In the first embodiment described above, although the guide wire 1 in which the gradient of the first expanded diameter portion 111 gradually changes along the longitudinal axis direction has been described, as shown in FIG. 3A, it is also possible to form a guide wire 1m1 provided with a core shaft 11m1 having a first expanded diameter portion 111m1, in which a plurality of truncated cones having different gradients are arranged along the longitudinal axis direction, with the end surfaces of the truncated cones joined so as to coincide with each other.

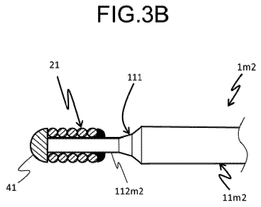

Furthermore, in the first embodiment described above, although the guide wire 1 in which the first extended portion 112 is arranged on the proximal end side of the first expanded diameter portion 111, and which extends toward the proximal end side in the longitudinal axis direction such that the distal end is continuous with the proximal end of the first expanded diameter portion 111 has been described, as shown in FIG. 3B, it is also possible to form a guide wire 1m2 provided with a core shaft 11m2, in which a first extended portion 112m2 is arranged on the distal end side of the first expanded diameter portion 111, and which extends toward the distal end side in the longitudinal axis direction such that the proximal end is continuous with the distal end of the first expanded diameter portion 111.

### [Second Embodiment]

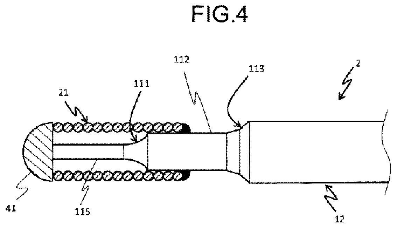

FIG. 4 is a partially enlarged schematic side view showing a second embodiment of the present invention. As illustrated in FIG. 4, a guide wire 2 is schematically configured by a core shaft 12, a first coil body 21, and a distal end fixing portion 41. The guide wire 2 differs from the first embodiment in that the core shaft 12 has a second expanded diameter portion 113. Since the configurations other than the configuration of the core shaft 12 are the same as those of the first embodiment, the same parts are designated by the same reference numerals and detailed description thereof will be omitted. Furthermore, the manner in which the guide wire 2 is used is the same as that of the first embodiment, and detailed description thereof will be omitted.

The core shaft 12 is a shaft in which the distal end portion has a diameter which reduces stepwise toward the distal end side. For example, the core shaft 12 has a first expanded diameter portion 111, a second expanded diameter portion 113, a first extended portion 112, and a small diameter portion 115.

The first expanded diameter portion 111 is a part of the core shaft 12 in which the diameter expands toward the proximal end side. The gradient of the proximal end of the first expanded diameter portion 111 is greater than the gradient of the distal end of the first expanded diameter portion 111, the average gradient from the distal end to the proximal end of the first expanded diameter portion 111 is greater than the gradient of the first extended portion 112, and the gradient of the first expanded diameter portion 111 increases continuously toward the proximal end side.

The second expanded diameter portion 113 is a part of the core shaft in which the diameter expands toward the proximal end side. For example, the second expanded diameter portion 113 is arranged on the proximal end side of the first extended portion 112 described below, and can be provided extending toward the proximal end side in the longitudinal axis direction such that the distal end is continuous with the proximal end of the first extended portion 112.

Here, it is preferable that the gradient of the second expanded diameter portion 113 is greater than the gradient of the first extended portion 112, and the gradient of the proximal end of the second expanded diameter portion 113 is greater than the gradient of the distal end of the second expanded diameter portion 113. An example of such a second expanded diameter portion 113 is a structure that uses truncated cones having different gradients, which are arranged along the longitudinal axis direction such that the truncated cone having the smaller gradient is on the distal end side of the second expanded diameter portion 113 and the truncated cone having the larger gradient is on the proximal end side of the second expanded diameter portion 113, and the truncated cones are joined such that the end surfaces coincide with each other. As a result, it is possible to increase the rigidity difference of the second expanded diameter portion 113 in the longitudinal axis direction compared to the first extended portion 112, and therefore, the proximal end portion K of the curved portion can be prevented from proceeding in multiple stages due to the large rigidity difference of the second expanded diameter portion 113 in addition to the first expanded diameter portion 111. Furthermore, because the gradient of the proximal end of the second expanded diameter portion 113 is greater than the gradient of the distal end of the second expanded diameter portion 113, plastic deformation of the second expanded diameter portion 113 can also be prevented.

Furthermore, it is preferable that the gradient of the second expanded diameter portion 113 is greater than the gradient of the first extended portion 112, and is constant along the longitudinal axis direction. As a result, it is possible to increase the rigidity difference of the second expanded diameter portion 113 in the longitudinal axis direction compared to the first extended portion 112, and therefore, the proximal end portion K of the curved portion can be prevented from proceeding proximally in multiple stages due to the large rigidity difference of the second expanded diameter portion 113 in addition to the first expanded diameter portion 111.

The first extended portion 112 is a part of the core shaft 12 which is adjacent to the first expanded diameter portion 111. For example, the first extended portion 112 can be formed having a cylindrical shape or a truncated cone shape that becomes thinner toward the distal end side, and provided so as to extend along the longitudinal axis direction. The first extended portion 112 is arranged between the first expanded diameter portion 111 and the second expanded diameter portion 113.

In the present embodiment, the core shaft 12 is configured such that the gradient of the first expanded diameter portion 111 gradually changes along the longitudinal axis direction (the outer peripheral surface 111a is curved), and the cylindrical small diameter portion 115 (constant outer diameter) is provided on the distal end of the first expanded diameter portion 111 so as to extend toward the distal end side. The second expanded diameter portion 113 uses two truncated cones having different gradients, which are arranged along the longitudinal axis direction such that the truncated cone having the smaller gradient is on the distal end side of the second expanded diameter portion 113 and the truncated cone having the larger gradient is on the proximal end side of the second expanded diameter portion 113, and the truncated cones are joined such that the end surfaces coincide with each other. The first extended portion 112 has a cylindrical shape extending along the longitudinal axis direction, and is arranged between the first expanded diameter portion 111 and the second expanded diameter portion 113 such that the distal end is adjacent to the proximal end of the first expanded diameter portion 111 and the proximal end is adjacent to the distal end of the second expanded diameter portion 113.

As a result of the guide wire 2 having the configuration described above, it is possible to prevent the proximal end portion of the U-shaped curved portion formed on the distal end portion of the guide wire 2 from proceeding beyond the first expanded diameter portion 111 to the proximal end side, and plastic deformation of the core shaft 12 at the first expanded diameter portion 111 can be prevented. Furthermore, because the guide wire 2 is provided with the second expanded diameter portion 113, even if the proximal end portion of the curved portion proceeds beyond the first expanded diameter portion 111 to the proximal end side, the position of the proximal end portion and the degree of curvature of the U-shaped curved portion can be adjusted in multiple stages according to a pushing force of the guide wire 2.

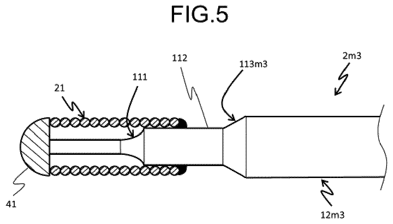

In the second embodiment described above, although the guide wire 2 provided with the core shaft 12 in which the gradient of the proximal end of the second expanded diameter portion 113 is greater than the gradient of the distal end of the second expanded diameter portion 113 has been described, as shown in FIG. 5, it is also possible to form a guide wire 2m3 which includes a core shaft 12m3 having a second expanded diameter portion 113m3, in which the gradient is constant over the entire longitudinal axis direction.

### [Third Embodiment]

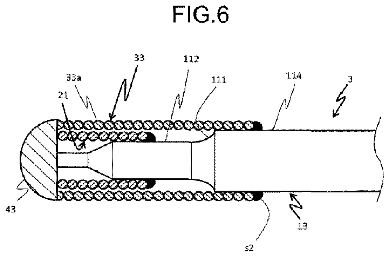

FIG. 6 is a partially enlarged schematic side view showing a third embodiment of the present invention. As illustrated in FIG. 6, a guide wire 3 is schematically composed of a core shaft 13, a first coil body 21, a second coil body 33, and a distal end fixing portion 43. The guide wire 3 differs from the first embodiment in that the core shaft 13, the second coil body 33, and the distal end fixing portion 43 are provided. Since the configurations other than the configurations of the core shaft 13, the second coil body 33, and the distal end fixing portion 43 are the same as those of the first embodiment, the same parts are designated by the same reference numerals and detailed description thereof will be omitted. Furthermore, the manner in which the guide wire 3 is used is the same as that of the first embodiment, and detailed description thereof will be omitted.

The core shaft 13 is a shaft in which the distal end portion has a diameter which reduces stepwise toward the distal end side. The core shaft 13 includes a first expanded diameter portion 111, a first extended portion 112, and a second extended portion 114.

The first expanded diameter portion 111 is a part of the core shaft 13 in which the diameter expands toward the proximal end side. The gradient of the proximal end of the first expanded diameter portion 111 is greater than the gradient of the distal end of the first expanded diameter portion 111, the average gradient from the distal end to the proximal end of the first expanded diameter portion 111 is greater than the gradient of the first extended portion 112, and the gradient of the first expanded diameter portion 111 increases continuously toward the proximal end side.

The first extended portion 112 is a part of the core shaft 13 arranged on the distal end side of the first expanded diameter portion 111. The first extended portion 112 can be configured to have a cylindrical shape or a truncated cone shape that becomes thinner toward the distal end side, and provided adjacent to the first expanded diameter portion 111 so as to extend along the longitudinal axis direction.

The second extended portion 114 is a part of the core shaft 13 arranged on the proximal end side of the first expanded diameter portion 111. The second extended portion 114 can be configured to have a cylindrical shape or a truncated cone shape that becomes thinner toward a distal end side, and provided so as to extend along the longitudinal axis direction.

In the present embodiment, the gradient of the first expanded diameter portion 111 gradually changes along the longitudinal axis direction, and the outer peripheral surface 111a of the first expanded diameter portion 111 is curved. The first extended portion 112 is cylindrical (constant outer diameter) and is arranged on the distal end side of the first expanded diameter portion 111, and is provided extending toward the distal end side in the longitudinal axis direction such that the proximal end is continuous with the distal end of the first expanded diameter portion 111. The second extended portion 114 is cylindrical (constant outer diameter) and is arranged on the proximal end side of the first expanded diameter portion 111, and is provided extending toward the proximal end side in the longitudinal axis direction such that the distal end is continuous with the proximal end of the first expanded diameter portion 111.

The second coil body 33 is a helical (coiled) member wound so as to cover the first coil body 21. The second coil body 33 may adopt, for example, a structure in which a wire 33a is wound to form a coil shape (see FIG. 6), or a structure in which a cylindrical member is subjected to slit processing or the like to form a coil shape (not illustrated). When the wire 33a is used as the second coil body 33, it is possible to use, for example, a wire equivalent to the wire 21a of the first coil body 21 described in the first embodiment as the wire 33a.

The proximal end of the second coil body 33 is not fixed to the first expanded diameter portion 111, but is fixed to the second extended portion 114 (joint part s2 in FIG. 6). On the other hand, the distal end of the second coil body 33 can, for example, be integrally fixed to the distal end fixing portion 43 together with the distal end of the core shaft 13 and the distal end of the first coil body 21.

As described above, because the guide wire 3 has the above configuration, even if the core shaft 13 breaks apart at the first expanded diameter portion 111, the distal end side and the proximal end side of the broken core shaft 13 are connected via the second coil body 33, and the distal end side and the proximal end side of the core shaft 13 can be prevented from separating.

Note that the present invention is not limited to the configurations of the embodiments described above, but is stipulated by claims, and the present invention is intended to include all modifications within the meaning and scope equivalent to those in claims.

### REFERENCE SIGNS LIST

- 1, 2, 3: Guide wire
- 11, 12, 13: Core shaft
- 21: First coil body
- 33: Second coil body
- 111: First expanded diameter portion
- 112: First extended portion
- 113: Second expanded diameter portion
- 114: Second extended portion
- 41, 43: Distal end fixing portion

## Claims

1. A guide wire comprising:
a core shaft having a first expanded diameter portion which expands in diameter toward a proximal end side, and a first extended portion having a cylindrical shape or a truncated cone shape that becomes thinner toward a distal end side, and which is provided adjacent to the first expanded diameter portion so as to extend along a longitudinal axis direction;
a first coil body wound so as to cover at least a portion of the core shaft; and
a distal end fixing portion at which a distal end of the core shaft and a distal end of the first coil body are fixed to each other; wherein
a gradient of a proximal end of the first expanded diameter portion is greater than a gradient of a distal end of the first expanded diameter portion,
an average gradient between a distal end and a proximal end of the first expanded diameter portion is greater than a gradient of the first extended portion, a gradient of the first expanded diameter portion increases continuously toward a proximal end side, and
a proximal end of the first coil body is not fixed to the first expanded diameter portion, but is fixed to the first extended portion.

2. The guide wire according to claim 1, wherein
the gradient of the first expanded diameter portion gradually changes along a longitudinal axis direction.

3. The guide wire according to claim 2, wherein
the gradient at the distal end of the first expanded diameter portion is zero, and the proximal end of the first expanded diameter portion stands perpendicular to a longitudinal axis.

4. The guide wire according to any one of claims 1 to 3, wherein
the core shaft includes a second expanded diameter portion which expands in diameter toward a proximal end side, and
the first extended portion is arranged between the first expanded diameter portion and the second expanded diameter portion.

5. The guide wire according to claim 4, wherein
a gradient of the second expanded diameter portion is greater than the gradient of the first extended portion, and
a gradient of the proximal end of the second expanded diameter portion is greater than a gradient of a distal end of the second expanded diameter portion.

6. The guide wire according to claim 4, wherein
a gradient of the second expanded diameter portion is greater than a gradient of the first extended portion, and is constant along a longitudinal axis direction.

7. The guide wire according to any one of claims 1 to 3, comprising:
a second coil body wound so as to cover the first coil body, wherein
the core shaft includes the first extended portion disposed on a distal end side of the first expanded diameter portion, and a second extended portion having a cylindrical shape or a truncated cone shape that becomes thinner toward a distal end side, and which is provided on a proximal end side of the first expanded diameter portion and extends along a longitudinal axis direction, and
a proximal end of the second coil body is not fixed to the first expanded diameter portion, but is fixed to the second extended portion.
